# EUROPÄISCHE PATENTSCHRIFT

(11) **EP 0 452 459 B1**
(45) Veröffentlichungstag und Bekanntmachung des Hinweises auf die Patenterteilung: **24.08.1994**
(21) Anmeldenummer: 90916721.5
(22) Anmeldetag: 29.10.1990
(51) Int. Cl.: B26B 21/00, B23K 26/00

(54) **RASIERAPPARAT**
RAZOR
RASOIR

(30) Priorität: 02.11.1989 DE 3936367
(43) Veröffentlichungstag der Anmeldung: 23.10.1991
(73) Patentinhaber: Philips Electronics N.V., 5621 BA Eindhoven (NL)
(72) Erfinder: Simon,Pal, D-4788 Warstein (DE)
(74) Vertreter: Bos, Kornelis Sjoerd
(86) Internationale Anmeldenummer: EP9001929
(87) Internationale Veröffentlichungsnummer: WO9106406

(56) Entgegenhaltungen:
- US-A- 4 819 669
- MAD, vol. 208, July 1979 Al Jaffee und John Wick : "The space age razor race", See page 38 "The laser razor".

## Beschreibung

Die Erfindung betrifft einen Rasierapparat mit einem Gehäuse und einer Scherplatte, die Haareintrittsöffnungen aufweist, und einer innerhalb des Gehäuses angeordneten Einrichtung zur Erzeugung eines Laserstrahls, der als Haarentferneinrichtung dient.

Ein derartiger Rasierapparat ist bekannt aus US-A-4 819 669.
Seit langem gibt es Hilfsgeräte, womit Haare von oder aus der Haut entfernt werden können. Die bekanntesten sind die Rasierapparate, sowohl Naßrasierer als auch Trockenrasierer. Diese haben alle den Nachteil, daß die Klingen beim Rasiervorgang nicht nur das Haar schneiden, sondern auch mit der Haut in Kontakt treten und somit die Haut mehr oder weniger reisen. Auch Epilationsgeräte um Haare zu entfernen, sind bekannt. Dies ist aber ein zeitraubender Prozeß. Aus US-A-4 819 669 ist ein Rasierapparat bekannt, wobei Haare durch ein Gitter in den Apparat hineintreten und mittels eines Laserstrahls versengt werden.

Der Erfindung liegt die Aufgabe zugrunde, einen Rasierapparat zu schaffen, bei dem der Laserstrahl die Haare an einer bestimmten Stelle erfaßt und durch verbrennen durchtrennt, ohne mit der Haut in Berührung zu kommen.

Die Aufgabe wird bei einem Rasierapparat der eingangs genannten Art dadurch gelöst, daß die Haareintrittsöffnungen einen Eintrittsspalt bilden und der Laserstrahl entlang des Eintrittsspaltes an der Innenseite der Scherplatte in einer verspiegelten Auftreffzone auftritt und dort das Haar erfaßt und durchtrennt.

Der Lasertechnologie macht es möglich einen Laserstrahl sehr genau zu richten. Bei Bewegung der Scherplatte über die Haut, treten die Haare in den Eintrittsspalt hinein und kommen über die entlang der Spaltkante angeordnete verspiegelte Auftreffzone zu liegen und werden dort von dem Laserstrahl erfaßt und durchtrennt. Es findet keine Berührung des Laserstrahls mit der sich in dem Eintrittsspalt wölbenden Hautfalte statt.

Vorzugsweise wird der von der verspiegelten Auftreffzone reflektierte Laserstrahl von einer an der Innenseite des Gehäuses angeordneten Fotozelle erfaßt, welche Fotozelle mit einer Unterbrechereinrichtung verbunden ist, mit deren Hilfe der Laserstrahl abschaltbar ist. Wenn unerwartet ein Hindernis in den Eintrittsspalt und in die Bahn des Laserstrahls tritt, wird dieser unterbrochen. Die Unterbrechung wird von der Fotozelle erfaßt und an die Unterbrechereinrichtung weitergeleitet, die den Laserstrahl abschaltet. Somit ist eine Sicherheitseinrichtung gegeben, die, insbesondere bei nicht sachgerechter Handhabung des Rasierapparats, in Aktion tritt. Das menschliche Haar ist so fein, daß die Fotozelle dieses nicht als Hindernis ansieht.

Da mit der Beseitigung der Haare durch Verbrennung mittels des Laserstrahls eine Geruchsentwicklung verbunden sein kann, kann im Gehäuse des Rasierapparats ein Geruchsfilter vorgesehen sein. Außerdem kann ein Ventilator im Gehäuse angeordnet sein, der die Abluft ansaugt, die dann durch den Geruchsfilter gereinigt wird. Die Breite des Eintrittsspalts liegt vorzugsweise zwischen etwa 0,2 mm und 1,0 mm, und liegt beispielsweise im Bereich von etwa 0,5 mm.

Der erzeugte Laserstrahl ist vorzugsweise flächig ausgebildet, wobei eine ebene Fläche mit minimaler Ausdehnung in senkrechter Richtung zur Fläche erzeugt wird, die entlang der Kante des Eintrittsspalts parallel zum Spalt ausgerichtet ist. Die Erzeugung eines solchen vorhangartigen Laserstrahls ist mittels der Lasertechnologie problemlos möglich. Die Wärmeleitung ermöglicht eine Kürzung der Haare auch über den Auftreffpunkt des Laserstrahls hinaus.

Vorzugsweise wird ein Laserstrahl erzeugt, dessen Frequenz der Absorptionsfrequenz der Haarsubstanz entspricht. Das Haar enthält Pigment, so daß der Laserstrahl im entsprechenden Frequenzbereich gut absorbiert wird. Diese Absorptionsfrequenz ist von derjenigen der wesentlichen Substanzen, aus denen die menschliche Haut aufgebaut ist, verschieden.

Im folgenden wird die vorliegende Erfindung unter Bezugnahme auf die beiliegenden Zeichnungen näher beschrieben. Dabei zeigen
- Fig. 1: einen schematisch vereinfachten Längsschnitt durch einen erfindungsgemäßen Rasierapparat;
- Fig. 2: einen vergrößerten Schnitt durch die Scherplatte;
- Fig. 3: eine prinzipielle Darstellung der Funktionsweise des erfindungsgemäßen Rasierapparats;
- Fig. 4: eine perspektivische Darstellung, aus der die Form des erzeugten Laserstrahls ersichtlich ist;
- Fig. 5: einen schematisch vereinfachten Längsschnitt durch einen Rasierapparat gemäß einer alternativen Ausführungsform.

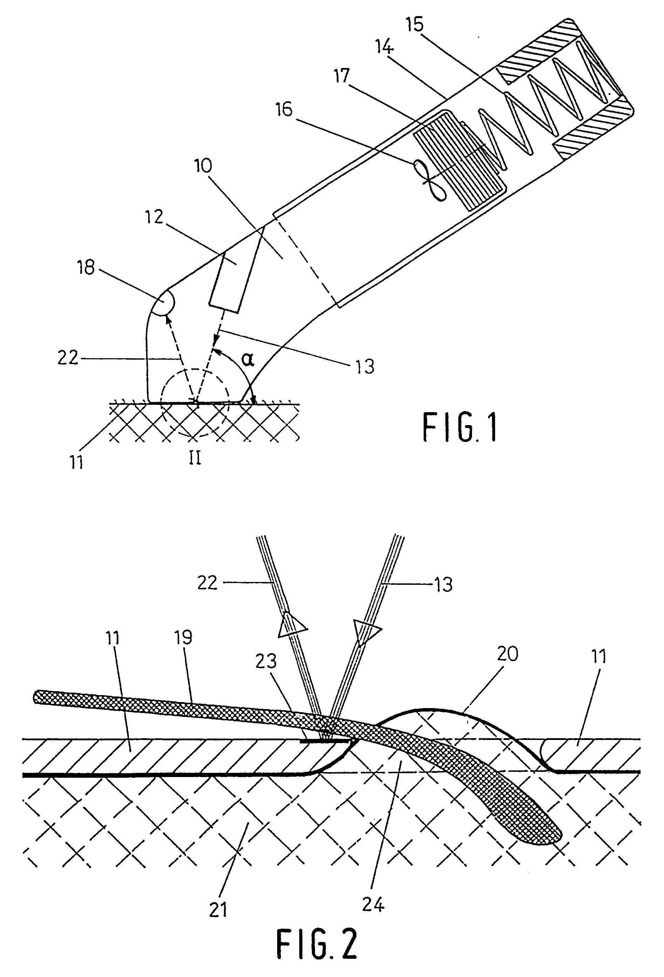

Zunächst wird auf die Fig. 1 Bezug genommen. Der erfindungsgemäße Rasierapparat weist ein Gehäuse mit einem unteren Gehäuseteil 10 und einem oberen Gehäuseteil 14 auf, wobei der obere Gehäuseteil 14 den unteren übergreift und auf diesem axial verschiebbar gegen die Kraft einer Feder 15 geführt ist. In dem Gehäuseteil 10 befindet sich im unteren Bereich an der oberen Gehäusewand die Einrichtung 12, die den Laserstrahl 13 erzeugt (Laserkanone). Die Laserkanone wird entweder aus einem Akkumulator gespeist oder über Netzstrom, wobei die Stromzufuhr in Fig. 1 nicht dargestellt ist. Im oberen Abschnitt des Gehäuseteils 10 befindet sich ein Ventilator, der die Abluft absaugt, sowie ein Geruchsfilter 17 oder Geruchsvernichter, wobei eine Ableitung der abgesaugten Luft vorgesehen sein kann, die in Fig. 1 nicht dargestellt ist. Wie aus Fig. 1 in Verbindung mit Fig. 2 hervorgeht, trifft der von der Laserkanone 12 erzeugte Laserstrahl 13 auf eine verspiegelte Zone 23 der Scherplatte 11 auf und wird dort reflektiert. Der reflektierte Strahl 22 trifft auf eine an der Gehäusewand befindliche Fotozelle 18 auf, die an eine Unterbrechereinrichtung angeschlossen ist. Das menschliche Haar ist so fein, daß die Fotozelle 18 dieses nicht als Hindernis ansieht, wenn es in den Strahlengang des Laserstrahls gerät.

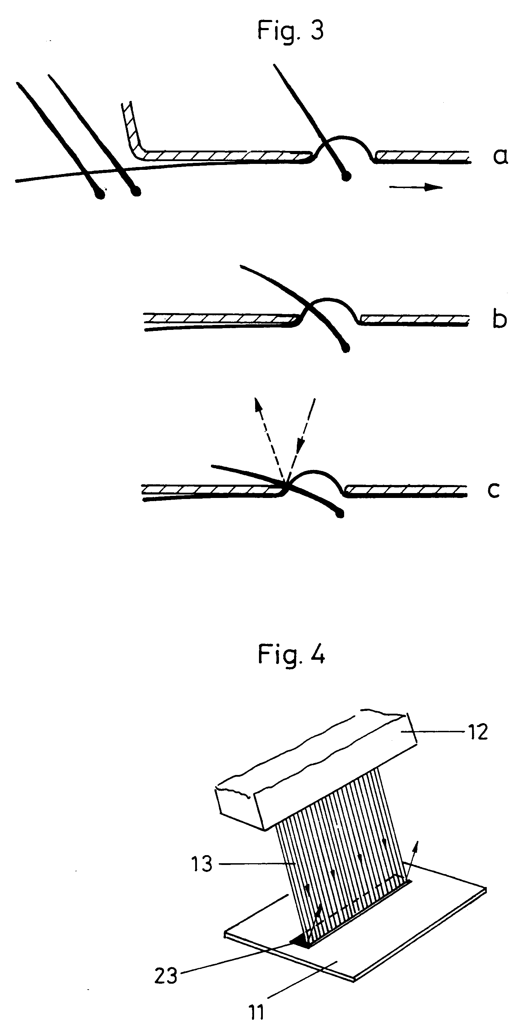

Es wird nun auf Fig. 2 Bezug genommen. Aus der Darstellung ist ersichtlich, daß die Scherplatte 11 auf der zu rasierenden Haut 20 aufgesetzt ist. In der Scherplatte 11 befindet sich ein sich senkrecht zur Zeichenebene erstreckender Spalt von zum Beispiel etwa 0,5 mm Breite. Da die Scherplatte 11 des Rasierers mit etwas Druck aufgesetzt wird und dann über die Haut bewegt wird, bildet das unter der Haut befindliche Gewebe 21 eine Falte oder Erhebung, die sich in den Spalt 24 der Scherplatte hineinwölbt. Die in diesem Hautsektor befindlichen Haare 19 ragen dann in das Gehäuseinnere. Ein solches Haar 19 ist in Fig. 2 zur Erläuterung dargestellt. Um den Schnitt des Haars mittels des erfindungsgemäßen Rasierers zu erläutern, wird auf die Figuren 2 und 3 Bezug genommen. Fig. 3 zeigt schematisch ein Haar in drei verschiedenen Positionen a, b und c bei Bewegung der Scherplatte über die Haut in der Zeichnung gesehen nach rechts. Aus der Darstellung geht hervor, daß sich das Haar in Skizze c bei Berührung mit der etwas spitz zulaufenden Kante des linken Rands des Spalts der Scherplatte innerhalb des Gehäuses des Rasierapparats befindet und einen vergleichsweise flachen Winkel mit der Scherplatte einnimmt. In dieser der Skizze c entsprechenden Lage wird das Haar nahe der Haarwurzel von dem Laserstrahl 13 (siehe Fig. 2) erfaßt, der auf der entlang der Spaltkante angeordneten verspiegelten Zone 23 auftrifft. Vorzugsweise sollte beim Schnitt der Laserstrahl einen steilen Winkel zum geschnittenen Haar einnehmen, im Idealfall wird das Haar von dem Laserstrahl im 90° Winkel durchtrennt. Wie weiterhin aus Fig. 2 hervorgeht, wird dabei zwar das Haar erfaßt, aber es findet keine Berührung des Laserstrahls mit der sich in den Spalt 24 wölbenden Hautfalte 20 statt. In entsprechender Weise werden alle in den Spalt der Scherplatte 11 hineinragenden Haare geschnitten. Fig. 4 zeigt die flächige Form des Laserstrahls 13, der etwa die Form eines Vorhangs hat und genau parallel zu dem Spalt 24 der Scherplatte ausgerichtet ist. Der Laserstrahl 13 bildet somit eine Ebene, die die Scherplatte 11 in der verspiegelten Zone 23 in einer scharfen Linie mit nur sehr geringer Breitenausdehnung schneidet.

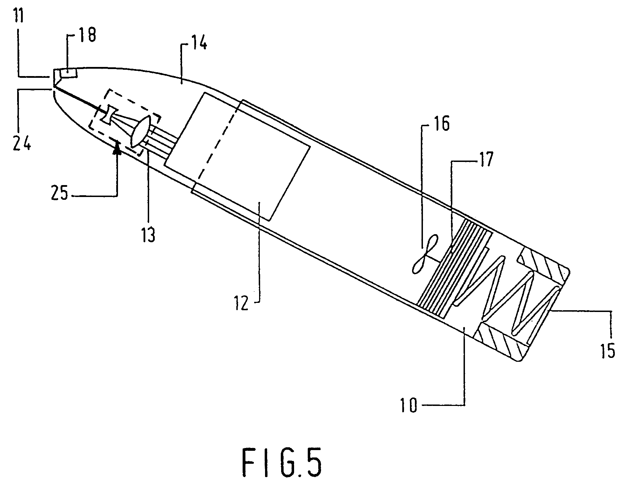

Bei der alternativen Ausführungsform gemäß Fig. 5 ist die Form des Gehäuses etwas anders ausgebildet und die Scherplatte ist schmaler. Außerdem ist eine Laseroptik 25 vorhanden, mittels derer der erzeugte Laserstrahl fokussiert und gerichtet wird, so daß er in gebündelter Form mit hoher Energie an der Innenseite der Scherplatte entlang des Eintrittsspalts auftrifft. Die Fotozelle ist im Gehäusekopf näher an der Auftrittszone des Laserstrahls angeordnet. Die Einrichtung zur Erzeugung des Laserstrahls ist axial im Gehäuse angeordnet und kann bei dieser Anordnung den Erfordernissen entsprechend groß dimensioniert werden.

## Patentansprüche

1. Rasierapparat mit einem Gehäuse (10) und einer Scherplatte (11), die Haareintrittsöffnungen aufweist, und einer innerhalb des Gehäuses (10) angeordneten Einrichtung (12) zur Erzeugung eines Laserstrahls (13), der als Schneideinrichtung dient, dadurch gekennzeichnet, daß die Haareintrittsöffnungen einen Eintrittsspalt (24) bilden und daß der Laserstrahl (13) entlang des Eintrittsspaltes (24) an der Innenseite der Scherplatte (11) in einer verspiegelten Auftreffzone (23) auftritt und dort das Haar erfaßt und durchtrennt.

2. Rasierapparat nach Anspruch 1, dadurch gekennzeichnet, daß der von der verspiegelten Auftreffzone (23) reflektierte Laserstrahl (22) von einer an der Innenseite des Gehäuses angeordneten Fotozelle (18) erfaßt wird, welche Fotozelle mit einer Unterbrechereinrichtung verbunden ist, mit deren Hilfe der Laserstahl (13) abschaltbar ist.

3. Rasierapparat nach Anspruch 1 oder 2, dadurch gekennzeichnet, daß der Auftreffwinkel α zwischen Laserstrahl (13) und Scherplatte (11) zwischen 50° und 85° beträgt.

4. Rasierapparat nach einem der Ansprüche 1 bis 3, dadurch gekennzeichnet, daß ein oberes Gehäuseteil (14) und ein unteres Gehäuseteil (10) vorgesehen sind, wobei das obere Gehäuseteil (14) das untere Gehäuseteil übergreift und das untere Gehäuseteil in dem oberen Gehäuseteil gegen die Kraft einer Feder (15) axial verschiebbar geführt ist.

5. Rasierapparat nach einem der Ansprüche 1 bis 4, dadurch gekennzeichnet, daß im Gehäuse ein Ventilator (16) angeordnet ist.

6. Rasierapparat nach Anspruch 5, dadurch gekennzeichnet, daß im Gehäuse eine Filtereinrichtung vorgesehen ist, durch die die vom Ventilator (16) in das Gehäuse gesaugte Abluft gefiltert wird.

7. Rasierapparat nach einem der Ansprüche 1 bis 6, dadurch gekennzeichnet, daß der erzeugte Laserstrahl (13) flächig ist und entlang einer zur Kante des Eintrittsspalts (24) parallelen Linie auf der Scherplatte (11) auftrifft.

8. Rasierapparat nach einem der Ansprüche 1 bis 7, dadurch gekennzeichnet, daß die Einrichtung (12) einen Laserstrahl erzeugt, dessen Frequenz in etwa der Absorptionsfrequenz der Haarsubstanz entspricht.

9. Rasierapparat nach einem der Ansprüche 1 bis 8, dadurch gekennzeichnet, daß eine Laseroptik (25) vorgesehen ist, durch die der erzeugte Laserstrahl gebündelt und / oder gerichtet wird.

## Claims

1. A shaving apparatus comprising as housing (10) and a shear plate (11) having hair-entry apertures and, arranged inside the housing (10), a device (12) for generating a laser beam (13) which acts as a cutting device, characterised in that hair-entry apertures form an entry slot (24) and in that the laser beam (13) impinges in a reflecting zone (23) of incidence along the entry slot (24) at the inner side of the shear plate (11) and acts upon and severs the hair at this location.

2. A shaving apparatus as claimed in Claim 1, characterised in that the laser beam (22) which is reflected from the reflecting zone (23) of incidence is detected by a photo-cell (18) arranged at the inner side of the housing, which photo-cell is connected to a switching device adapted to turn off the laser beam (13).

3. A shaving apparatus as claimed in Claim 1 or 2, characterised in that the angle of incidence α between the laser beam (13) and the shear plate (11) is between 50° and 85°.

4. A shaving apparatus as claimed in any one of the Claims 1 to 3, characterised in that there have been provided an upper housing section (14) and a lower housing section (10), the upper housing section (14) engaging over the lower housing section and the lower housing section being guided in the upper housing section so as to be axially movable against the force of a spring (15).

5. A shaving apparatus as claimed in any one of the Claims 1 to 4, characterised in that a fan (16) has been arranged in the housing.

6. A shaving apparatus as claimed in Claim 5, characterised in that in the housing there has been provided a filter device to filter the air drawn into the housing by the fan (16).

7. A shaving apparatus as claimed in any one of the Claims 1 to 6, characterised in that the generated laser beam (13) is planar and impinges on the shear plate (11) along a line parallel to the edge of the entry slot (24).

8. A shaving apparatus as claimed in any one of the Claims 1 to 7, characterised in that the device (12) generates a laser beam whose frequency substantially corresponds to the absorption frequency of the hair substance.

9. A shaving apparatus as claimed in any one of the Claims 1 to 8, characterised in that there has been provided an optical system by means of which the generated laser beam is focused and/or directed.

## Revendications

1. Rasoir comportant un boîtier (10) et un peigne (11) percé d'ouvertures d'entrée de poil, ainsi qu'un dispositif (12), disposé à l'intérieur du boîtier (10), pour engendrer un rayon laser (13) servant d'organe de coupe, caractérisé en ce que les ouvertures d'entrée de poil forment une fente d'entrée (24) et en ce que le faisceau laser (13), à travers la fente d'entrée (24), frappe la face intérieure du peigne (11) dans une zone d'incidence réfléchissante (23) pour y attaquer le poil et le couper.

2. Rasoir selon la revendication 1, caractérisé en ce que le faisceau laser (22) réfléchi par la zone d'incidence réfléchissante (23) est détecté par une cellule photo-électrique (18) disposée sur la face intérieure du boîtier et qui est reliée à un dispositif interrupteur permettant d'éteindre le faisceau laser (13).

3. Rasoir selon la revendication 1 ou 2, caractérisé en ce que l'angle d'incidence α que fait le faisceau laser (13) avec le peigne (11) est compris entre 50° et 85°.

4. Rasoir selon l'une des revendications 1 à 3, caractérisé en ce qu'il est prévu une partie supérieure de boîtier (14) et une partie inférieure de boîtier (10), la partie supérieure de boîtier (14) enjambant sur la partie inférieure de boîtier et la partie inférieure de boîtier étant guidée axialement dans la partie supérieure de boîtier, à l'encontre de la force d'un ressort (15).

5. Rasoir selon l'une des revendications 1 à 4, caractérisé en ce qu'un ventilateur (16) est placé dans le boîtier.

6. Rasoir selon la revendication 5, caractérisé en ce que, dans le boîtier, est prévu un dispositif de filtrage servant à filtrer l'air aspiré dans le boîtier par le ventilateur (16).

7. Rasoir selon l'une des revendications 1 à 6, caractérisé en ce que le faisceau laser engendré (13) est de forme plate et frappe le peigne (11) suivant une ligne parallèle au bord de la fente d'entrée (24).

8. Rasoir selon l'une des revendications 1 à 7, caractérisé a ce que le dispositif (12) engendre un faisceau laser dont la fréquence correspond à peu près à la fréquence d'absorption de la substance de poil.

9. Rasoir selon l'une des revendications 1 à 8, caractérisé en ce qu'il est prévu un système optique laser (25) servant à focaliser et/ou diriger le faisceau laser engendré.
